# FASCICULE DE BREVET EUROPEEN

(11) **EP 0 467 766 B1**
(45) Date de publication et mention de la délivrance du brevet: **29.05.1996**
(21) Numéro de dépôt: 91401965.8
(22) Date de dépôt: 15.07.1991
(51) Int. Cl.: A61N 1/36, A61H 39/00

(54) **Appareil d'électro-stimulations**
Elektroreizgerät
Electrostimulation apparatus

(30) Priorité: 18.07.1990 FR 9009136
(43) Date de publication de la demande: 22.01.1992
(73) Titulaire: LABORATOIRES DEGLAUDE (Sociéte anonyme), F-92220 Bagneux (FR)
(72) Inventeur: Privas, Yves, Pompano Beach 33062 Florida (US)
(74) Mandataire: Pinguet, André

(56) Documents cités:
- EP-A- 0 145 176
- EP-A- 0 275 213
- DE-A- 3 202 010
- FR-A- 2 418 646
- FR-A- 2 441 294
- FR-A- 2 506 612
- US-A- 4 112 923

## Description

La présente invention concerne un appareil d'éléctro-stimulation, particulièrement destiné à induire les fumeurs à la désaccoutumance du tabac.

De nombreuses méthodes ont été expérimentées dans le domaine de la désaccoutumance du tabac. On peut citer par exemple les médicaments à base de lobéline, de quinine, de nicotine, et les systèmes tels que l'acuponcture ou de stimulation auriculaire obtenue par l'implantation d'une agraphe ou d'aiguilles ou encore par stimulation électrique.

La pose d'une agraphe présente l'inconvénient de devoir être réalisée par un spécialiste. Elle peut présenter une gêne. Elle réalise une stimulation permanente qui n'est pas nécessaire.

L'implantation d'aiguilles est une méthode délicate, qui doit être réalisée par un spécialiste dans des conditions d'hygiène draconiennes.

La stimulation électrique présente un certain nombre d'avantages puisqu'il n'est pas indispensable d'avoir recours à un spécialiste, l'effet est puissant et rapide, l'hygiène est complète, la stimulation peut intervenir chaque fois que le besoin se fait sentir.

Les appareils de stimulation électrique actuellement connus et utilisés dans l'état de l'art sont principalement des appareils fixes dotés d'une alimentation sur le secteur et dont l'utilisation est réservée aux professionnels.

D'autres appareils, transportables, peuvent être quant à eux alimentés par une source d'énergie renouvelable. Certains se composent d'un boîtier, connecté à un crayon de contact par l'intermédiaire d'un cordon, d'autres comportent une pointe de contact.

Lorsqu'un utilisateur les met en oeuvre par application sur un nombre variable de points de stimulation dont la cartographie est pécisée, le crayon ou la pointe de contact transmettent une série d'impulsions éléctriques, ressenties par le système nerveux comme des fourmillements.

Mais ces appareils ne sont pas toujours efficaces; leur fonctionnement peut être pertubé par un certain nombre de facteurs tant au niveau de la connectique de la liaison appareil/cordon lorsque tel est le cas, qu'au niveau plus général de l'instabilité de certaines grandeurs électriques provoquée par la variation du niveau de charge de la source d'énergie renouvelable. Ainsi, il est connu des documents FR-A-2 418 646 et US-A-4,112,923 des appareils d'électro-stimulation comportant des moyens pour détecter une chute de tension de la source d'énergie et d'en prévenir l'utilisateur.

Le demandeur a récemment découvert que la stabilisation des dites grandeurs électriques à des valeurs constantes, en particulier pour la fréquence et l'intensité de pointe, ceci quel que soit le niveau de charge de la source d'énergie dont les spécifications constituent par ailleurs un élément indissociable de l'ensemble, améliore grandement l'efficacité des dispositifs de stimulation électrique du genre en question.

La présente invention a pour objet un appareil d'électro-stimulation mettant en oeuvre cette découverte, et présentant d'autres caractéristiques avantageuses décrites ci-après.

La présente invention a donc pour objet un appareil d'électro-stimulation constitué d'un boîtier autonome comportant au moins une source d'énergie, une extrémité de contact destinée à être appliquée sur la peau d'un utilisateur, de façon à transmettre à la peau une série de décharges électriques répétitives, caractérisé en ce que l'appareil comporte des moyens pour stabiliser au moins un paramètre caractérisant la série de décharges. Lesdits moyens pour stabiliser au moins un paramètre caractérisant la série de décharges peuvent stabiliser l'intensité électrique maximale des décharges et/ou la fréquence des décharges. L'appareil peut comporter un premier moyen de signalisation pour signaler la marche de l'appareil. L'appareil peut aussi comporter un deuxième moyen de signalisation pour signaler une usure de la source d'énergie telle que l'intensité des décharges ne puisse plus être maintenue constante. Avantageusement, l'appareil comporte aussi des moyens pour arrêter l'émission des décharges électriques en cas de détection d'une usure de la source d'énergie telle que l'intensité des décharges ne puisse plus être maintenue constante.

Avantageusement, le boîtier est fermé de façon à ne pas pouvoir être ouvert par un utilisateur, de façon à ce qu'il ne puisse pas remplacer la source d'énergie après que l'émission des décharges électriques a été arrêtée, suite à la détection d'une usure de la source d'énergie telle que l'intensité des décharges ne puisse plus être maintenue constante.

Le boîtier peut contenir un circuit électronique comprenant une alimentation connectée à un oscillateur et à un circuit de détection de tension basse, lui-même connecté audit deuxième moyen de signalisation et à l'oscillateur de façon que lorsque le circuit de détection de tension basse détecte une tension de l'alimentation inférieure à un certain seuil, ledit circuit de détection de tension basse envoie un signal à l'oscillateur pour bloquer le fonctionnement dudit oscillateur, et provoque l'émission d'un signal de tension basse par ledit deuxième moyen de signalisation, ledit oscillateur étant connecté à un circuit de commande pour amplifier un signal périodique en sortie de l'oscillateur, ledit circuit de commande étant lui-même connecté audit premier moyen de signalisation, pour émettre un signal de marche de l'appareil, et ledit circuit de commande étant aussi connecté à un circuit de conversion de tension pour élever la tension du signal amplifié par le circuit de commande, ledit circuit de conversion étant doté d'un circuit de réglage de l'intensité des décharges électriques et connecté à une électrode s'étendant jusqu'à l'extrémité de contract.

Avantageusement, le premier moyen de signalisation et le deuxième moyen de signalisation consistent en une diode électroluminescente bicolore pouvant s'éclairer de deux couleurs différentes, une première couleur signalant une tension d'alimentation trop basse et une deuxième couleur signalant la marche de l'appareil.

D'autres caractéristiques et avantages de l'invention apparaîtront au cours de la description détaillée qui suit, donnée à titre d'exemple non limitatif, en regard des dessins joints, sur lesquels :
- la figure 1 est une vue de côté du boîtier d'une forme préférée de réalisation de l'invention,
- la figure 2 est une vue de dessus du boîtier de la figure 1,
- la figure 3 est une vue de côté du boîtier de la figure 1, ouvert, montrant une première face de la plaque support du circuit électronique de l'appareil,
- la figure 4 est une vue en coupe selon la ligne IV-IV du boîtier de la figure 1,
- la figure 5 est une vue de la deuxième face de la plaque support de la figure 3, à une échelle légèrement réduite,
- la figure 6 est un schéma synoptique du circuit électronique de la forme de réalisation de l'invention des figures 1 à 5, et
- la figure 7 est un schéma détaillé du circuit électronique de la figure 6.

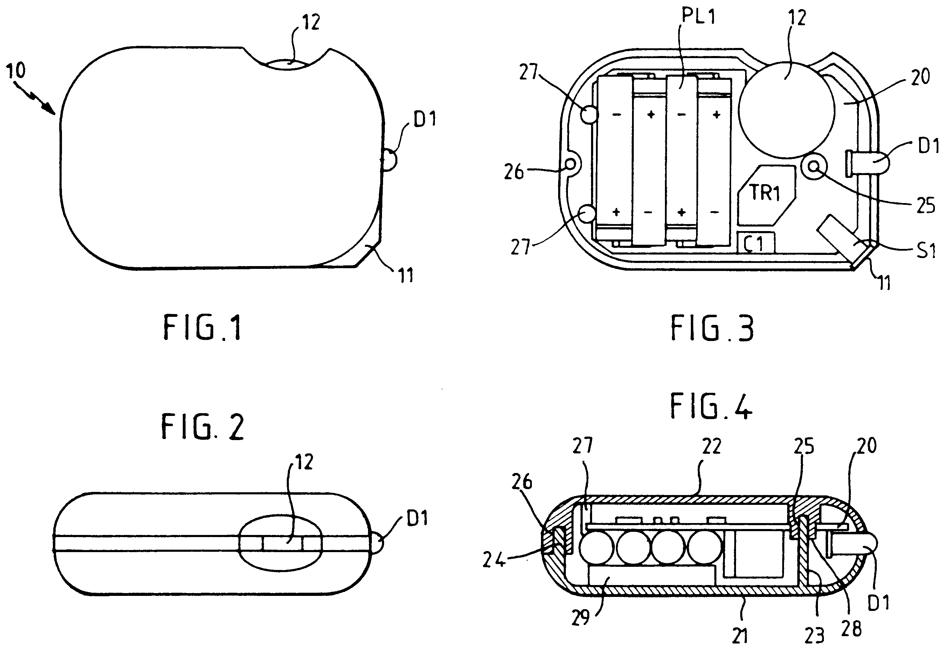

Les figures 1 et 2 présentent la forme extérieure d'un exemple d'appareil d'électro-stimulation selon l'invention. Celui-ci a la forme d'un boîtier applati 10, de taille à être tenu à la main, présentant deux faces ayant sensiblement la forme de rectangles à coins arrondis. A partir de l'un des coins arrondis est réalisée une extrémité de contact 11. Le boîtier comporte en outre une molette de réglage 12 et un voyant, de préférence une diode électroluminescente D1 bicolore, qui peut s'éclairer en vert ou en rouge.

Lorsqu'un utilisateur ressent le besoin de fumer, il applique l'extrémité de contact 11 en un point de sa peau, de préférence sur une oreille, et tourne la molette 12. La rotation de la molette 12 a pour effet de déclencher l'émission d'impulsions électriques par l'extrémité de contact 11, et de régler l'intensité de pointe desdites impulsions. La marche de l'appareil est visualisée par une lumière verte clignotante émise par la diode électroluminescente D1.

Dans cet exemple, le boîtier a une longueur de 8 cm, une largeur de 5,2 cm et une épaisseur de 2,4 cm. Il peut donc facilement être glissé dans une poche de vêtement, et ainsi rester à portée de la main de l'utilisateur pour être disponible au moment où il ressent le besoin de fumer.

L'appareil d'électro-stimulation fonctionne à l'aide de piles. Lorsque les piles sont suffisamment usées pour que l'intensité des décharges ne puisse plus être maintenue constante par l'appareil, la diode électroluminescente D1 affiche une couleur rouge continue et l'émission des impulsions électriques est coupée. L'appareil doit alors être jeté.

En effet, le boîtier 10 ne peut pas être ouvert sans être endommagé, ce qui empêche l'utilisateur de changer les piles. Ceci évite que l'utilisateur ne remplace les piles usagées par des piles différentes, qui pourraient avoir des caractéristiques électriques suffisamment différentes de celles des piles initiales pour compromettre l'efficacité de l'appareil.

En effet, il est indispensable, pour que l'appareil donne des décharges électriques d'intensité suffisante, que les piles aient une résistance interne faible. Les piles pourront par exemple être du type alcalines manganèse, mais des piles zinc-carbone de même tension nominale ne pourraient pas convenir.

Les figures 3 et 4 montrent l'agencement intérieur du boîtier 10. Le boîtier se divise en deux coques 21 et 22, qui sont assemblées l'une à l'autre de façon à ne pas pouvoir être séparées par un utilisateur. Par exemple, la coque 21 peut comporter deux tenons 23 et 24 adaptés à s'emboîter dans deux mortaises respectives 25 et 26 réalisées sur la coque 22. Tenons et mortaises sont fixés définitivement par collage.

A l'intérieur du boîtier 10 est fixée une plaque support 20, portant différents composants électroniques, et sur laquelle est imprimé un circuit électrique. La plaque support peut être fixée par tout moyen connu. Par exemple, la coque 22 comporte deux bras élastiques 27. Chaque bras 27 s'étend à partir de la coque 22 jusqu'à une extrémité présentant une encoche dans laquelle un bord de la plaque 20 est adapté à s'encliqueter. De plus, la plaque 20 est percée d'un trou et la coque 22 comporte un ergot 28 présentant un épaulement, ledit ergot étant adapté à s'emboîter dans le trou de la plaque 20. La fixation de la plaque 20 se fait donc par emboîtement du trou de la plaque 20 sur l'ergot 28, jusqu'en butée dudit épaulement, et par encliquetage simultané sur les bras élastiques 27.

La plaque 20 comporte deux faces, dont une première face est représentée à la figure 3. Sur cette première face sont fixées les piles PL1, ici quatre piles alcalines au manganèse de 1,5 V, connectées en série, la molette 12, la diode électroluminescente D1, un transformateur TR1 et un condensateur C1 dont le rôle sera précisé ci-dessous, et une électrode de sortie S1 s'étendant jusqu'à l'extrémité de contact 11. Les coques 21 et 22 comportent des évidements pour laisser le passage à la molette 12, à la diode électroluminescente D1 et à l'électrode S1. La coque 23 comporte en outre une nervure 29 destinée à maintenir en place les piles PL1.

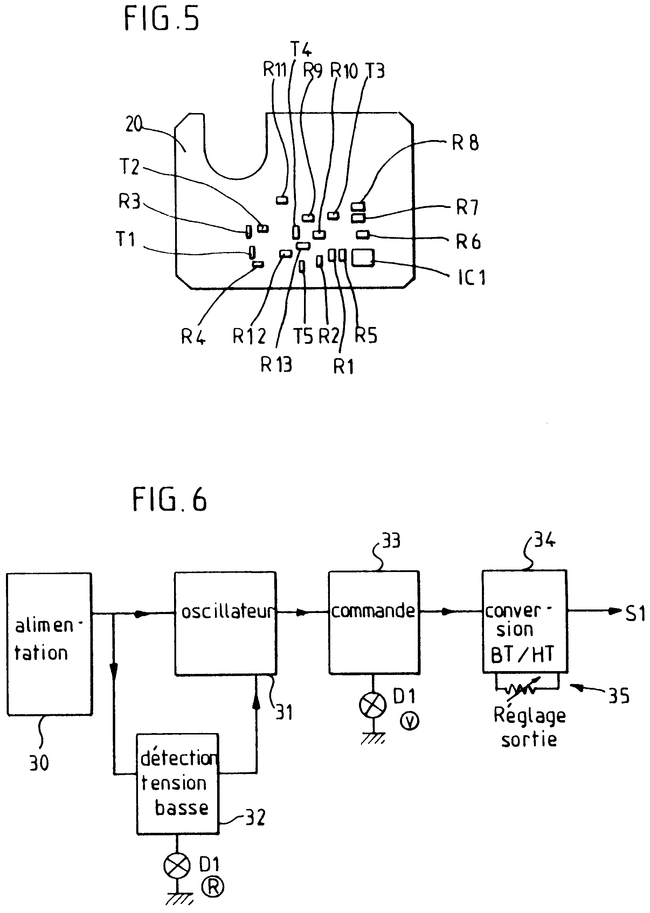

Une deuxième face de la plaque support 20 est représentée à la figure 5. Sur cette face est imprimé un circuit électrique (non représenté) auquel sont connectés divers composants électroniques qui seront dits en détail ci-après.

La figure 6 est un schéma synoptique du circuit électronique de la forme de réalisation de l'invention des figures 1 à 5. Ce circuit comporte une alimentation électrique 30, connectée à un oscillateur 31 et à un circuit de détection de tension basse 32, lui-même connecté à l'oscillateur 31 et à la diode électroluminescente D1. Lorsque le circuit 32 détecte une tension inférieure à un certain seuil en sortie de l'alimentation 30, il provoque l'allumage de la couleur rouge de la diode électroluminescente bicolore D1 et transmet à l'oscillateur 31 un signal de tension basse.

L'oscillateur 31 est connecté à un circuit de commande 33 ; tant que l'oscillateur ne reçoit pas ledit signal de tension basse, il transmet au circuit de commande 33 des impulsions électriques, à une fréquence comprise entre 3 et 8 Hz par exemple. Lorsqu'il reçoit ledit signal de tension basse, l'oscillateur 31 ne transmet pas d'impulsion électrique au circuit de commande 33. Le circuit de commande 33 est connecté à la diode électroluminescente D1 et au circuit de conversion BT/HT 34. Lorsqu'il reçoit des impulsions, le circuit de commande 33 transmet au circuit 34 une série d'impulsions de plus grande puissance électrique, et provoque l'allumage intermittent de la couleur verte de la diode électroluminescente bicolore D1. Le circuit de conversion 34 élève la tension des impulsions électriques, par exemple jusqu'à 500 V, et les transmet à l'électrode de sortie 31. De plus, le circuit 34 est connecté à un circuit 35 permettant de régler l'intensité électrique des impulsions émises par l'électrode S1.

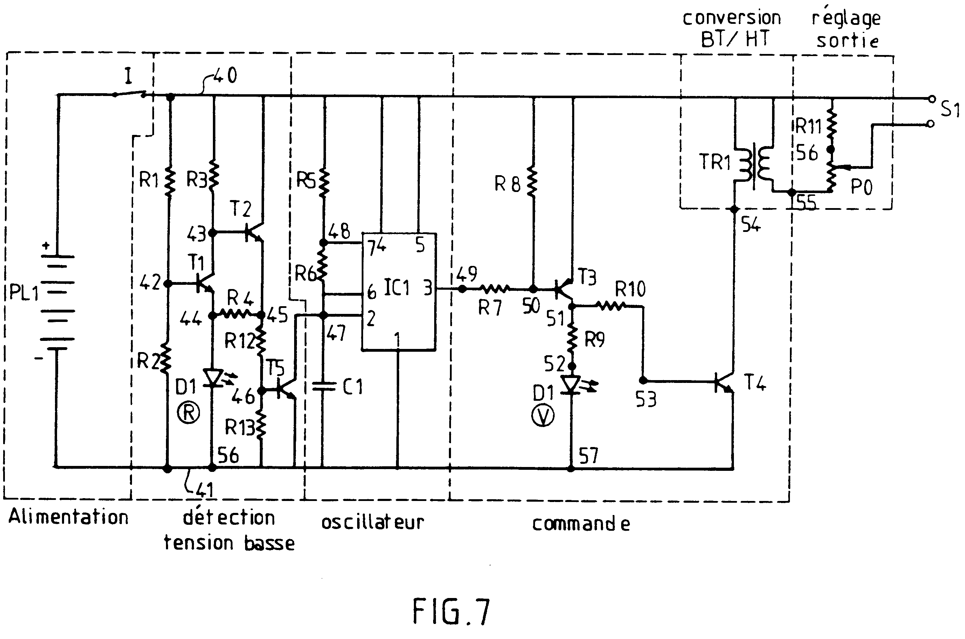

La figure 7 présente un schéma détaillé du circuit électronique de la figure 6. Le circuit d'alimentation est constitué de quatre piles PL1 de 1,5 V de type alcalines-manganèse montées en série, de façon à produire une tension nominale de 6 V. Un interrupteur I est monté en série avec les piles PL1. Avantageusement, l'interrupteur est un contact de fin de course de la molette 12.

Du circuit d'alimentation partent deux lignes électriques 40 et 41. Entre les deux lignes 40 et 41 est tout d'abord connecté le circuit de détection de tension base qui se compose :
- de deux résistances R1 et R2 montées en diviseur de tension, la résistance R1 étant connectée à la ligne 40, la résistance R2 étant connectée à la résistance R1 en un point 42 et à la ligne 41. Dans cet exemple R1 a une valeur de 33 kΩ et R2 de 43 kΩ ;
- d'un premier transistor T1, de type NPN, dont la base est connectée au point 42;
- d'une résistance R3, ici de valeur 15 kΩ, connectée au collecteur du transistor T1 en un point 43, et à la ligne 40;
- d'un transistor T2, de type NPN, dont la base est connectée au point 43 et le collecteur à la ligne 40;
- d'une résistance R4, ici de valeur 33 kΩ, connecté entre un point 44 relié à l'émetteur du transistor T1 et un point 45 relié à l'émetteur du transistor T2;
- de la diode électroluminescente bicolore D1, dont une borne permettant son éclairage en rouge est connecté au point 44, et une autre borne est connectée à la ligne 41, en un point 56 ;
- de deux résistances R12 et R13 montées en diviseur de tension. Dans cet exemple, la résistance R12 vaut 10 kΩ et la résistance R13 vaut 3,8 kΩ. La résistance R12 est connectée au point 45, et la résistance R13 est connectée à la résistance R12 en un point 46, et à la ligne 41 ;
- d'un transistor T5 de type NPN dont la base est connectée au point 46, l'émetteur à la ligne 41, et le collecteur est connecté en un point 47 du circuit oscillateur.

Le circuit oscillateur comporte:
- un multivibrateur astable IC1 doté de sept bornes de connexion, dont deux bornes 4 et 5 sont connectées à la ligne 40, une borne 1 est connectée à la ligne 41, et une borne de sortie 3 est connectée à un point 49 du circuit de commande,
- une résistance R5 connectée entre la ligne 40 et un point 48 relié à une borne 7 du multivibrateur IC1 ; la résistance R5 a ici une valeur de 160 kΩ;
- une résistance R6 connectée à deux bornes 2 et 6 du multivibrateur IC1 ; la résistance R6 a ici une valeur de 47 kΩ;
- un condensateur C1, ici de capacité 1 µF, connecté entre le point 47 et la ligne 41.

Le circuit de commande comprend:
- une résistance R7, ici de valeur 10 kΩ, connectée au point 49 ;
- une résistance R8, ici de valeur 10 kΩ, connectée à la résistance R7 en un point 50 et à la ligne 40 ;
- un transistor T3, de type PNP, dont la base est connectée au point 50 et l'émetteur est connecté à la ligne 40 ;
- une résistance R9, ici de valeur 100Ω , connectée en un point 51 au collecteur du transistor T3;
- la diode électroluminescente D1 bicolore, dont une borne permettant son éclairage en vert est connectée à la résistance R9 en un point 52, et dont une autre borne est connectée à la ligne 41 en un point 57;
- la résistance R10, ici de valeur 410Ω connectée au point 51;
- le transistor T4, de type NPN, dont la base est connectée à la résistance R10 en un point 53, dont l'émetteur est connecté à la ligne 41, et dont le collecteur est connecté au circuit de conversion de tension en un point 54.

Le circuit de conversion de tension comprend un transformateur TR1 doté d'un enroulement primaire et d'un enroulement secondaire. L'enroulement primaire est connecté entre la ligne 40 et le point 54, tandis que l'enroulement secondaire est connecté entre la ligne 40 et un point 55 commun avec le circuit de réglage de sortie. La tension entre le point 55 et la ligne 40 est ici de 500 V.

Le circuit de réglage de la sortie, qui permet de régler l'intensité de pointe des impulsions électriques émises par l'électrode S1, comprend :
- une résistance R11, de valeur 220 kΩ, connectée entre la ligne 40 et un point 56,
- un potentiomètre Po actionné par la molette 12, connecté entre le point 55 et le point 56, ledit potentiomètre comportant une sortie connectée à une borne de l'électrode S1, une autre borne de l'électrode S1 étant connectée à la ligne 40.

Lorsque l'interrupteur I est fermé et que les piles PL1 délivrent une tension supérieure à une tension limite, par exemple 4,2 V, il circule un courant faible dans les résistances R4, R12 et R13, de telle sorte que la tension entre le point 44 et la ligne 41 est trop faible pour provoquer l'allumage de la diode D1 en rouge. De plus, du fait du faible courant circulant dans R13, le potentiel du point 46 n'est pas suffisant pour rendre le transistor T5 saturé; le condensateur C1 n'est donc pas en court-circuit.

Le multivibrateur IC1, associé aux résistances R5, R6 et au condensateur C1, délivre un signal de faible puissance à sa sortie 3, sous la forme d'un signal de tension périodique en créneaux, de fréquence par exemple égale à 5 Hz. Si Vo est la tension produite par les piles PL1, la différence de potentiel entre la ligne 41 et la sortie 3 varie périodiquement entre + Vo et - Vo, à la fréquence de 5 Hz dans cet exemple particulier.

Lorsque la différence de potentiel entre la ligne 41 et la sortie 3 est de + Vo, la sortie 3 est au même potentiel que la ligne 40. Aucun courant ne passe donc dans les résistances R8 et R7. Le point 50 relié à la base du transistor T3 est donc au même potentiel que la ligne 40, reliée à l'émetteur du transistor T3. Aucun courant ne peut donc passer entre l'émetteur et le collecteur du transistor T3. Aucun courant ne passe donc entre les points 52 et 57 ; la diode électroluminescente ne s'éclaire pas en vert. Comme le point 53 se trouve alors au même potentiel que la ligne 41, le transistor T4 est dans un état bloqué, de sorte que l'enroulement primaire du tranformateur TR1 n'est parcouru par aucun courant. Aucune tension n'est donc délivrée aux bornes de l'enroulement secondaire du transformateur TR1, et donc aucun courant n'est délivré en sortie de l'électrode S1 en contact avec la peau de l'utilisateur.

Lorsque la différence de potentiel entre la sortie 3 du multivibrateur et la ligne 41 est de - Vo, un courant circule dans les résistances R8 et R7, en direction de ladite sortie 3. Le point 50, relié à la base du transistor T3, est donc à un potentiel inférieur à la ligne 40, reliée à l'émetteur du transistor T3. Le transistor T3 devient alors passant, de sorte qu'un courant le traverse, entre l'émetteur et le collecteur. Un courant traverse donc la résistance R9 et la diode électroluminescente D1 entre les points 52 et 57, de sorte que ladite diode s'éclaire en vert. Le point 51, et donc le point 53 relié à la base du transistor T4, sont donc à un potentiel supérieur à la ligne 41, reliée à l'émetteur du transistor T4, qui devient alors passant. Un courant traverse alors le transistor T4 entre le collecteur et l'émetteur, en traversant l'enroulement primaire du transformateur TR1. Ce courant a une valeur imposée par le courant parcourant le transistor T4 de la base vers l'émetteur, qui dépend de la résistance R10.

Le transformateur TR1 élève la tension, de sorte qu'il délivre entre la ligne 40 et le point 55 des impulsions de tension de 500 V, à la fréquence de 5 Hz dans cet exemple particulier.

Ces impulsions de tension sont transformées en impulsions de courant traversant le circuit formé par la résistance R11, le potentiomètre Po et la peau de l'utilisateur comprise entre les bornes de l'électrode S1.

La valeur maximale du courant de sortie, traversant la peau de l'utilisateur comprise entre les bornes de l'électrode S1, est fixée par le potentiomètre Po, actionné par la molette 12.

Lorsque la tension aux bornes des piles PL1 devient inférieure à 4,2 V, le transistor T1 devient saturé. Le potentiel du point 44 augmente donc suffisamment pour allumer la diode D1 en rouge, et, du fait du courant plus important traversant la résistance R13, le potentiel du point 46 augmente suffisamment pour rendre saturé le transistor T5, qui met en court-circuit le condensateur C1, ce qui arrête l'oscillateur, dont la sortie 3 reste à la tension + Vo. L'appareil ne peut donc plus délivrer d'impulsions électriques en sortie de l'électrode S1, et doit alors être jeté.

La tension de 4,2 V correspond à une tension de chaque pile de 1,05 V. Cette tension limite ne correspond donc pas à une usure totale des piles, où la tension aux bornes de chaque pile se situe plutôt près de 0,8 V, mais correspond à une usure des piles telle que l'intensif des décharges électriques ne puisse plus être maintenue constante. A ce niveau d'usure, une moitié seulement de l'énergie normalement libérable par les piles a été consommée, mais la résistance interne des piles, qui augmente lorsque la tension aux bornes des piles diminue, devient trop élevée pour que l'intensité électrique nécessaire pour assurer l'efficacité de la stimulation soit fournie.

## Revendications

1. Appareil d'électro-stimulation constitué d'un boîtier (10) autonome comportant au moins une source d'énergie (PL1), une extrémité de contact (11) destinée à être appliquée sur la peau d'un utilisateur, de façon à transmettre à la peau une série de décharges électriques répétitives, un circuit de détection de tension basse (32), pour détecter une chute de tension de la source d'énergie (PL1) en dessous d'une valeur prédéterminée, caractérisé en ce que ledit circuit de détection de tension basse (32) est adapté à arrêter l'émission des décharges électriques en cas de détection de ladite chute de tension de la source d'énergie (PL1) au-dessous de ladite valeur prédéterminée de façon que l'appareil cesse de fonctionner dès que l'intensité des décharges électriques ne peut plus être maintenue constante.

2. Appareil d'électro-stimulation selon la revendication 1, caractérisé en outre en ce que le boîtier (10) est fermé de façon à ne pas pouvoir être ouvert par un utilisateur, de façon à ce qu'il ne puisse pas remplacer la source d'énergie, après que l'émission des décharges électriques a été arrêtée suite à la détection d'une chute de tension de la source d'énergie (PL1) au-dessous de ladite valeur prédéterminée.

3. Appareil d'électro-stimulation selon la revendication 1 ou la revendication 2, caractérisé en outre en ce qu'il comporte un moyen (D1) de signalisation de la marche de l'appareil.

4. Appareil d'électro-stimulation selon l'une quelconque des revendications précédentes, caractérisé en outre en ce qu'il comporte un moyen (D1) de signalisation de tension basse pour signaler une chute de tension de la source d'énergie (PL1) au-dessous de ladite valeur prédéterminée.

5. Appareil d'électro-stimulation selon la revendication 1 ou la revendication 2, caractérisé en outre en ce que le boîtier (10) contient un circuit électronique comprenant une alimentation (30) connectée à un oscillateur (31) et audit circuit de détection de tension basse (32), lui-même connecté à un moyen de signalisation de tension basse (D1) et à l'oscillateur (31), de façon que lorsque le circuit de détection de tension basse (32) détecte une tension de l'alimentation inférieure à un certain seuil, ledit circuit de détection de tension basse envoie un signal à l'oscillateur pour bloquer le fonctionnement dudit oscillateur, et provoque l'émission d'un signal de tension basse par ledit moyen de signalisation de tension basse (D1), ledit oscillateur (31) étant connecté à un circuit de commande (33) pour amplifier un signal périodique en sortie de l'oscillateur, ledit circuit de commande (33) étant lui-même connecté à un moyen de signalisation de la marche de l'appareil (D1), et ledit circuit de commande (33) étant aussi connecté à un circuit de conversion de tension (34) pour élever la tension du signal amplifié par le circuit de commande (33), ledit circuit de conversion (34) étant doté d'un circuit de réglage (35) de l'intensité des décharges électriques et connecté à une électrode (31) s'étendant jusqu'à l'extrémité de contract (11).

6. Appareil d'électro-stimulation selon la revendication 5, caractérisé en outre en ce que le moyen de signalisation de marche et le moyen de signalisation de tension basse consistent en une diode électroluminescente bicolore (D1) pouvant s'éclairer de deux couleurs différentes, une première couleur signalant une tension d'alimentation trop basse et une deuxième couleur signalant la marche de l'appareil.

## Claims

1. Electro-stimulation apparatus comprising a self-contained housing (10) containing at least one energy source (PL1) and a contact end (11) for application against the skin of a user so as to transmit a series of repetitive electrical discharges to the skin, a low voltage detection circuit (32) for detecting a drop in the voltage from the energy source (PL1) to below a predetermined value, characterized in that said low voltage detection circuit (32) is adapted to stop electrical discharges being transmitted in the event of said drop in the voltage of the energy source (PL1) below said predetermined value being detected so as to prevent the apparatus from operating as soon as the electrical discharge currents can no longer be kept constant.

2. Electro-stimulation apparatus according to claim 1, further characterized in that the housing (10) is closed so as to prevent it being opened by a user, thereby preventing the user replacing the energy source after electrical discharging has been stopped following the detection of the voltage from the energy source (PL1) dropping below said predetermined value.

3. An electro-stimulation apparatus according to claim 1 or claim 2, further characterized in that it includes indicator means (D1) for indicating that the apparatus is working.

4. Electro-stimulation apparatus according to any preceding claim, further characterized in that it includes "voltage low" indicator means (D1) for indicating that the voltage from the energy source (PL1) has dropped below said predetermined value.

5. Electro-stimulation apparatus according to claim 1 or claim 2, further characterized in that the housing (10) contains an electronic circuit comprising a power supply (30) connected to an oscillator (31) and to said low voltage detection circuit (32), itself connected to "voltage low" indicator means (D1) and to the oscillator (31) in such a manner that when the low voltage detection circuit (32) detects a power supply voltage lower than a certain threshold, said low voltage detection circuit applies a signal to the oscillator to stop said oscillator operating and also causes a "voltage low" signal to be emitted by said "voltage low" indicator means (D1), said oscillator (31) being connected to a control circuit (33) to amplify a periodic output signal from the oscillator, said control circuit (33) itself being connected to indicator means (D1) for indicating that the apparatus is working, and said control circuit (33) also being connected to a voltage converter circuit (34) for raising the voltage of the signal amplified by the control circuit (33), said converter circuit (34) being provided with an adjustment circuit (35) for adjusting the electrical discharge current and connected to an electrode (31) extending to the contact end (11).

6. An electro-stimulation apparatus according to claim 5, further characterized in that the means for indicating that the apparatus is working and the "voltage low" indicator means are both constituted by a single two-color light-emitting diode (D1) capable of emitting two different colors: a first color indicating a too low power supply voltage, and a second color indicating that the apparatus is working.

## Patentansprüche

1. Elektrostimulationsgerät, welches aus einem autonomen Gehäuse (10) besteht, dieses umfassend wenigstens eine Energiequelle (PL 1), ein Kontaktende (11), welches dazu bestimmt ist, auf die Haut eines Benutzers aufgesetzt zu werden, um auf die Haut eine Reihe von wiederholten elektrischen Entladungen zu übertragen, einen Niederspannungserfassungsschaltkreis (32), um einen Spannungsabfall der Energiequelle (PL 1) unter einen vorgegebenen Wert zu erfassen, dadurch **gekennzeichnet,** daß der genannte Niederspannungserfassungsschaltkreis (32) dazu ausgelegt ist, die Abgabe von elektrischen Entladungen im Falle der Erfassung des genannten Spannungsabfalles der Energiequelle (PL 1) unter den vorgegebenen Wert zu beenden derart, daß das Gerät seine Funktion einstellt, sobald die Intensität der elektrischen Entladungen nicht mehr konstant gehalten werden kann.

2. Elektrostimulationsgerät nach Anspruch 1, weiter dadurch **gekennzeichnet,** daß das Gehäuse (10) so geschlossen ist, daß es nicht von einem Benutzer geöffnet werden kann, so daß er die Energiequelle nicht ersetzen kann, nachdem die Abgabe von elektrischen Entladungen infolge des Erfassens eines Spannungsabfalles der Energiequelle (PL 1) unter den vorgegebenen Wert beendet worden ist.

3. Elektrostimulationsgerät nach Anspruch 1 oder Anspruch 2, weiter dadurch **gekennzeichnet,** daß es Mittel (D1) zum Signalisieren des Betriebes des Gerätes umfaßt.

4. Elektrostimulationsgerät nach einem der vorangehenden Ansprüche, weiter dadurch **gekennzeichnet,** daß es Niederspannungssignalisierungsmittel (D1) zum Signalisieren eines Spannungsabfalles der Energiequelle (PL 1) unter den genannten vorgegebenen Wert umfaßt.

5. Elektrostimulationsgerät nach Anspruch 1 oder Anspruch 2, weiter dadurch **gekennzeichnet,** daß das Gehäuse (10) einen elektronischen Schaltkreis enthält, welcher eine mit einem Oszillator (31) und mit dem genannten Niederspannungserfassungsschaltkreis (32) verbundene Versorgung (30) umfaßt, wobei dieser selbst mit Niederspannungssignalisierungsmitteln (D1) und mit dem Oszillator (31) verbunden ist derart, daß dann, wenn der Niederspannungserfassungsschaltkreis (32) eine Versorgungsspannung feststellt, die unter einer bestimmten Schwelle liegt, dieser Niederspannungserfassungsschaltkreis an den Oszillator ein Signal schickt, um den Betrieb dieses Oszillators zu sperren, und die Ausgabe eines Niederspannungssignals durch die Niederspannungssignalisierungsmittel (D1) veranlaßt, wobei der Oszillator (31) mit einem Steuerschaltkreis (33) zum Verstärken eines periodischen Signals am Ausgang des Oszillators verbunden ist, und wobei dieser Steuerschaltkreis (33) selbst mit Mitteln (D1) zum Signalisieren des Betriebes des Gerätes verbunden ist, und wobei der Steuerschaltkreis (33) außerdem mit einem Spannungswandlerschaltkreis (34) verbunden ist, um die Spannung des vom Steuerschaltkreis (33) verstärkten Signals anzuheben, und wobei der Wandlerschaltkreis (34) mit einem Schaltkreis (35) zum Regeln der Intensität der elektrischen Entladungen ausgestattet und mit einer Elektrode (31) verbunden ist, die sich bis zum Kontaktende (11) hin erstreckt.

6. Elektrostimulationsgerät nach Anspruch 5, weiter dadurch **gekennzeichnet,** daß die Mittel zum Signalisieren eines Betriebes und die Mittel zum Signalisieren einer Niederspannung aus einer zweifarbigen Elektrolumineszenz-Elektrode (D1) bestehen, die in zwei unterschiedlichen Farben leuchten kann, wobei eine erste Farbe eine zu niedrige Versorgungsspannung und eine zweite Farbe den Betrieb des Gerätes signalisiert.
